(19) Europäisches Patentamt
European Patent Office
Office européen des brevets

(11) EP 0 712 846 B1

(12) **FASCICULE DE BREVET EUROPEEN**

(45) Date de publication et mention de la délivrance du brevet:
**11.08.1999 Bulletin 1999/32**

(51) Int Cl.[6]: **C07D 233/32**, C07D 239/10, C07D 243/04, C07D 245/02

(21) Numéro de dépôt: **95402556.5**

(22) Date de dépôt: **15.11.1995**

(54) **Procédé de préparation de (méth)acrylates d'alkylimidazolidone**

Verfahren zur Herstellung von Alkylimidazolidonmethacrylaten

Process of preparation of alkylimidazolidone methacrylates

(84) Etats contractants désignés:
**AT BE CH DE DK ES FR GB GR IE IT LI LU NL PT SE**

(30) Priorité: **18.11.1994 FR 9413848**

(43) Date de publication de la demande:
**22.05.1996 Bulletin 1996/21**

(73) Titulaire: **ELF ATOCHEM S.A.**
**92800 Puteaux, Hauts-de-Seine (FR)**

(72) Inventeurs:
- **Riondel, Alain**
  **F-57600 Forbach (FR)**
- **Herbst, Gilles**
  **F-57350 Spicheren (FR)**
- **Levray, André**
  **F-57500 Saint Avold (FR)**

(74) Mandataire: **Chaillot, Geneviève**
**Cabinet CHAILLOT,**
**16-20, avenue de L'Agent Sarre,**
**B.P. 74**
**92703 Colombes Cédex (FR)**

(56) Documents cités:
**EP-A- 0 236 994**
**EP-A- 0 433 135**
**EP-A- 0 453 638**
**EP-A- 0 571 851**
**EP-A- 0 619 309**
**EP-A- 0 650 962**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 712 846 B1

## Description

La présente invention porte sur un procédé de fabrication d'un composé de formule:

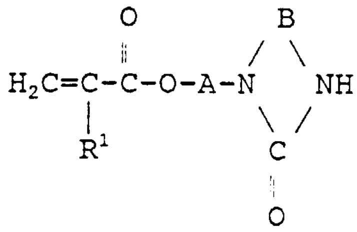

(I)

dans laquelle :

- $R^1$ représente hydrogène ou méthyle ; et
- A et B représentent chacun indépendamment une chaîne hydrocarbonée droite ou ramifiée, ayant de 2 à 5 atomes de carbone,

par réaction d'un (méth)acrylate de formule :

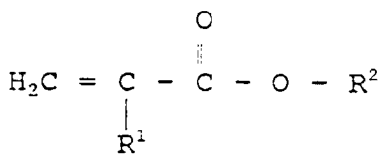

(II)

dans laquelle :

- $R^1$ a la signification précitée ; et
- $R^2$ représente un groupe alkyle en $C_1$-$C_4$, avec un alcool hétérocyclique de formule :

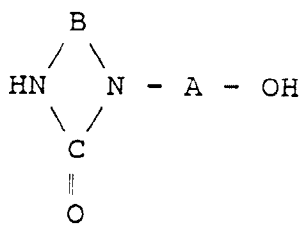

(III)

dans laquelle A et B ont les significations précitées.

Ces composés de formule (I) sont connus pour leur rôle dans la constitution de polymères utiles comme revêtements et adhésifs, pour le traitement du papier et des textiles, notamment par le brevet américain US-A-2 871 223, ainsi que pour leurs utilisations comme agents de traitement du cuir, et dans la production de peintures en émulsion. Le méthacrylate d'éthylimidazolidone (MEIO) est principalement utilisé dans les peintures en tant que promoteur d'adhésion humide.

Il est connu, par la demande de brevet européen EP-A-O 433 135, d'utiliser, comme catalyseurs pour cette réaction, les dialkyloxydes d'étain, les dialkyldialcoxydes d'étain et les dialkyldiesters d'étain. On cite, entre autres, le di-n-butyloxyde d'étain (DBTO).

Cependant, dans le cas de la synthèse du MEIO, on cherche à atteindre une conversion la plus importante possible de l'hydroxyéthylimidazolidone (HEIO), ce qui, dans le cas d'une catalyse par le DBTO, nécessite un niveau thermique élevé.

On a donc recherché un catalyseur autre permettant notamment d'obtenir une productivité normale à des températures de réaction plus basses.

La Société déposante a ainsi découvert que l'utilisation d'un chélate de calcium avec un composé 1,3-dicarbonyle, en particulier l'acétylacétonate de calcium ($Ca(acac)_2$), ou d'un tel chélate en mélange avec au moins l'un parmi les dialkyloxydes, dialkyldialcoxydes et dialkylesters d'étain, permet d'opérer à une température inférieure à 100°C (95°C-96°C notamment), tout en conduisant à des résultats comparables du point de vue du rendement en MEIO et de la conversion de l'HEIO. Ceci a fait l'objet de la demande de brevet français FR-A-2 703 682.

La Société déposante a parallèlement découvert que l'utilisation d'un alcoolate de magnésium permet d'atteindre les mêmes résultats (demande de brevet français FR-A-2 711 653 du 27 octobre 1993).

Recherchant à améliorer encore l'activité catalytique, la Société déposante a maintenant découvert que l'association d'un alcoolate de magnésium avec un autre catalyseur choisi parmi les chélates de calcium précités et les composés de l'étain précités, permet une catalyse avec une cinétique beaucoup plus rapide que celle observée dans le meilleur des cas avec l'alcoolate de magnésium seul.

La présente invention a donc pour objet le procédé de fabrication d'un composé de formule (I), tel qu'il a été défini ci-dessus, en présence d'un catalyseur constitué par un mélange formé (a) d'au moins un alcoolate de magnésium et (b) d'un composant choisi parmi les chélates de calcium avec les composés 1,3-dicarbonylés, les dialkyloxydes d'étain, les dialkylalcoxydes d'étain et les dialkyldiesters d'étain.

A titre d'exemples d'alcoolates de magnésium $Mg(OR)_2$, on peut citer ceux pour lesquels R représente un groupe alkyle linéaire en $C_1$-$C_4$, tel que méthyle, éthyle, n-propyle, n-butyle. On peut citer plus particulièrement les alcoolates pour lesquels R représente éthyle ou n-propyle.

On préfère utiliser le diéthylate de magnésium comme constituant (a) du catalyseur utile selon l'invention.

A titre d'exemples de composés dicarbonylés, on peut mentionner un ester d'acide β-cétonique, comme l'ester acétylacétique, ou une 1,3-dicétone, comme l'acétylacétone, la 3-méthylacétylacétone, la benzoylacétone, le dibenzoylméthane, la 2,4-hexanedione, la 3,5-heptanedione, la 3-phényl acétylacétone, la 4,4,4-trifluoro-1-phényl-1,3-butanedione, la 2,2,6,6-tétraméthyl-3,5-heptanedione, la 1,1,1-trifluoro-5,5-diméthyl-2,4-hexanedione et la 1,1,1-trifluoro-2,4-pentanedione. En particulier, on peut citer l'acétylacétonate de calcium comme composant (b) du catalyseur utile selon l'invention.

Comme dialkyloxyde d'étain entrant dans la définition du constituant (b) du catalyseur, on cite, en particulier, le di-n-butyloxyde d'étain (DBTO).

Comme exemples de réactifs de formule (II), on peut citer notamment les acrylates et méthacrylates de méthyle, d'éthyle, de n-propyle, de n-butyle et d'isobutyle.

Comme exemple d'alcool hétérocyclique de formule (III), on peut citer, notamment, la 1-(2-hydroxyéthyl)-imidazolidyl-2-one (HEIO).

Pour la mise en oeuvre du procédé selon l'invention, on utilise le constituant (a) du catalyseur dans une quantité généralement comprise entre 0,5 et 4% environ en moles, et, de préférence, entre 1 et 2,5% environ en moles, et le constituant (b), dans une quantité généralement comprise entre 0,01 et 2% environ en moles, et, de préférence, entre 0,02 et 1% environ en moles, les quantités étant données par rapport à l'alcool hétérocyclique de formule (III).

La réaction du procédé selon l'invention peut être effectuée en présence d'un excès de l'un ou l'autre des réactifs. Il est toutefois conseillé que le rapport molaire (méth)acrylate de formule (II)/alcool hétérocyclique de formule (III) soit compris entre 1,1 et 7,0 environ, de préférence entre 2,0 et 6,0. En opérant avec un large excès molaire de (méth)acrylate par rapport à l'alcool hétérocyclique, on obtient, à l'issue de la réaction, une solution de composé de formule (I) dans le (méth)acrylate qui peut être utilisée directement pour certaines applications, telles que l'obtention de peintures et revêtements ou bien le traitement du cuir.

La réaction du procédé selon l'invention est, de préférence, effectuée en présence d'au moins un inhibiteur de polymérisation, utilisé, par exemple, à raison de 0,05 à 0,5% en poids sur la base du poids de l'alcool hétérocyclique de formule (III). Comme exemples d'inhibiteurs de polymérisation utilisables, on peut citer notamment la phénothiazine, l'éther méthylique de l'hydroquinone, le di-tertiobutylcatéchol, l'hydroquinone, le p-anilinophénol, la paraphénylène diamine et leurs mélanges en toutes proportions.

La réaction du procédé selon l'invention est effectuée, de préférence, sous une pression ne dépassant pas la pression atmosphérique, par exemple une pression comprise entre 0,3 et 1 bar. De façon avantageuse, la réaction est réalisée sous bullage d'air pour améliorer l'efficacité des stabilisants. Elle est effectuée en mélangeant le (méth) acrylate de formule (II) et l'alcool hétérocyclique de formule (III), et en chauffant le mélange réactionnel au reflux, généralement à une température comprise entre 75 et 105°C, cette température étant évidemment dépendante de la nature exacte de l'alcool et du (méth)acrylate, et du système catalytique mis en oeuvre.

Dans la mise en oeuvre du procédé selon l'invention, il est conseillé d'atteindre une déshydratation maximale avant l'addition du catalyseur, de manière à éviter la désactivation de ce dernier par l'eau. On peut parvenir à ce résultat, par exemple, en chauffant le mélange initial de (méth)acrylate de formule (II), d'alcool hétérocyclique de formule (III) et d'inhibiteur de polymérisation au reflux, tout en séparant par distillation l'azéotrope de (méth) acrylate et d'eau lorsqu'il se forme un azéotrope de méthacrylate et d'eau. A ce stade, après séparation du distillat, le catalyseur est introduit dans le mélange réactionnel à chaud.

La durée de la réaction selon l'invention, dépend évidemment de conditions réactionnelles, telles que la température, la pression et la quantité de catalyseur utilisée, mais est généralement comprise entre 3 et 15 heures environ. Elle dépend évidemment aussi de la nature des réactifs mis en oeuvre.

Le mélange réactionnel est donc chauffé au reflux jusqu'à ce que la température de tête atteigne la température de distillation de l'azéotrope du (méth)acrylate et de l'alcool de formule $R_2OH$ formé par la réaction lorsqu'il se forme un azéotrope.

L'excès éventuel de (méth)acrylate peut ensuite être éliminé par évaporation, de manière à isoler le composé de formule (I) du milieu réactionnel, généralement à l'état solide : ainsi, l'acrylate de 1-(2-hydroxyéthyl) imidazolidyl-2-one est un solide blanc cristallin de température de fusion égale à 43°C, soluble à froid dans les cétones, les alcools, les hydrocarbures aromatiques et l'eau, insoluble à froid dans les hydrocarbures saturés et qui précipite à 0°C dans l'acrylate d'éthyle. Le méthacrylate de 1-(2-hydroxyéthyl) imidazolidyl-2-one est un solide blanc cristallin de température de fusion égale à 47°C, possédant les mêmes propriétés de solubilité que l'acrylate précédent. A l'issue de l'opération d'évaporation, le produit solide cristallin peut en outre être purifié par filtration puis lavage à l'aide d'éther de pétrole, et séchage.

L'isolement du composé (I) peut aussi être réalisé par évaporation partielle du (méth)acrylate, puis cristallisation à une température suffisamment basse (de préférence inférieure ou égale à 0°C) et pendant une durée suffisamment longue (pouvant atteindre jusqu'à 15 heures), puis filtration suivie des étapes de purification décrites ci-dessus.

Enfin, une troisième méthode pour isoler le composé de formule (I) de la solution le contenant, consiste à effectuer une extraction par l'eau, suivie d'une décantation, d'une concentration du (méth)acrylate et des étapes de purification décrites ci-dessus.

Les exemples suivants illustrent l'invention. Dans ces exemples, les pourcentages sont indiqués en poids sauf indication contraire.

<u>EXEMPLES 1 ET 2 (COMPARATIFS), 3 (DE L'INVENTION), 4 (COMPARATIF) ET 5 (DE L'INVENTION)</u>

<u>Mode opératoire général</u> :

Dans un réacteur en verre, à double enveloppe, équipé d'une sonde de mesure de la température, d'un agitateur mécanique à vitesse variable, d'une colonne adiabatique à garnissage, surmontée d'une tête de reflux, on introduit 221 g d'HEIO et 635 g de méthacrylate de méthyle (MAM), ainsi que 0,4 g de phénothiazine (PTZ) en tant que stabilisant. On met en service une stabilisation en tête de colonne par une solution à 0,1% d'éther méthylique de l'hydroquinone (EMHQ) dans le MAM. On porte le contenu du réacteur à ébullition sous pression atmosphérique pendant 1 heure, à une température de tête de colonne de 98-100°C et une température de pied de colonne inférieure ou égale à 100°C, et on élimine l'eau par distillation azéotropique avec le méthacrylate de méthyle.

Ensuite, on introduit dans le réacteur le (ou les) catalyseur(s) en mettant en oeuvre la quantité indiquée, ainsi que la quantité nécessaire de MAM pour obtenir un rapport molaire MAM/HEIO égal à 3,5. On ajuste la pression pour maintenir dans le réacteur une température de 95°C. On régule le soutirage de l'azéotrope MAM/MeOH par une température de consigne en tête de colonne (égale à 63°C). Lorsque la quantité de méthanol soutirée correspond à la quantité attendue, on prolonge la réaction jusqu'à ce que l'on ne constate plus de formation de méthanol (température en tête de colonne = température d'ébullition du MAM), à reflux total, sous la pression considérée.

Après refroidissement, on récupère du MEIO brut.

Le rendement en MEIO et la conversion de l'HEIO sont déterminés d'après l'analyse par chromatographie en phase liquide (HPLC) du brut réactionnel, par les équations suivantes :

$$\text{Conversion C HEIO \%} = \frac{(\text{HEIO de départ - HEIO final})}{\text{HEIO de départ}} \times 100$$

$$\text{Rendement R MEIO \%} = \frac{\text{Nombre de moles de MEIO formé}}{\text{Nombre de moles d'HEIO de départ}} \times 100$$

Les résultats des différents essais effectués sont rapportés dans les Tableaux 1 et 2 ci-après. Dans ces Tableaux, on a rapporté la conversion C de l'HEIO et le rendement R du MEIO.

Tableau 1

| Exemple | Catalyseur et % molaire de catalyseur par rapport à HEIO | Durée (h) | Analyse HPLC du mélange brut obtenu (%) | | | C (%) | R (%) |
|---|---|---|---|---|---|---|---|
| | | | MAM | HEIO | MEIO | | |
| 1 (comparatif) | $(EtO)_2Mg$ (2) | 7 | 47,4 | 0,3 | 42,5 | 99,2 | 73 |
| 2 (comparatif) | DBTO (0,23) | 6 | 60,1 | 11,7 | 23,4 | 59,2 | 53,4 |
| 3 | $(EtO)_2Mg$ (2) + DBTO (0,23) | 5,5 | 49 | 0,84 | 39,5 | 95,5 | 81 |
| 4 (comparatif) | $Ca(acac)_2$ (0,04) | 6 | 55,8 | 10,3 | 31,2 | 69,9 | 59,9 |
| 5 | $(EtO)_2Mg$ (2)+ $Ca(acac)_2$ (0,04) | 4,5 | 30,5 | 1,1 | 53,3 | 97,6 | 75 |

Tableau 2

| Catalyseur et % molaire de catalyseur par rapport à HEIO | C (%) | | | |
|---|---|---|---|---|
| | 1 h | 2 h | 3 h | 4 h |
| $(EtO)_2Mg$ (2) | | 31 | 43 | 63 |
| DBTO (0,23) | | 21 | 23 | 26 |
| $(EtO)_2Mg$ (2) + DBTO (0,23) | | 52 (calculé) 62 | 66 (calculé) 89 | 89 (calculé) 100 |
| $(EtO)_2Mg$ (2) | 14 | 32 | 43 | |
| $Ca(acac)_2$ (0,04) | 18 | 32 | 43 | |
| $(EtO)_2Mg$ (2) + $Ca(acac)_2$ (0,04) | 32 (calculé) 75 | 64 (calculé) 85 | 86 (calculé) 95 | |

C (%) : Conversion en fonction du temps

Les résultats rassemblés dans le Tableau 1 montrent une activité catalytique des systèmes à deux composants des Exemples 3 et 5 supérieure à celle des composants catalytiques pris séparément.

Cette forte activité se traduit par une diminution significative de la durée de réaction. Elle ne résulte pas d'une simple addition des performances catalytiques des composants individuels de ces systèmes, en terme de conversion de l'HEIO, mais d'une synergie entre lesdits composants individuels.

En effet, en examinant les résultats du Tableau 2, on s'aperçoit que la conversion théorique de l'HEIO au cours des quatre premières heures de la réaction, résultant de l'addition calculée des conversions obtenues avec chaque composant catalytique individuel, est inférieure à celle donnée par les systèmes catalytiques de l'invention.

## Revendications

**1.** Procédé de fabrication d'un composé de formule:

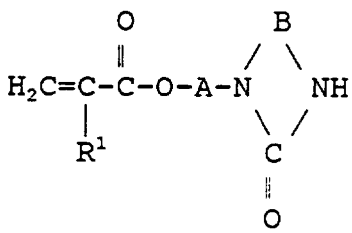

(I)

dans laquelle :

- $R^1$ représente hydrogène ou méthyle ;
- A et B représentent chacun indépendamment une chaîne hydrocarbonée droite ou ramifiée, ayant de 2 à 5 atomes de carbone,

par réaction d'un (méth)acrylate de formule :

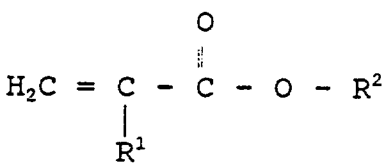

(II)

dans laquelle :

- $R^1$ a la signification précitée ; et
- $R^2$ représente un groupe alkyle en $C_1$-$C_4$, avec un alcool hétérocyclique de formule :

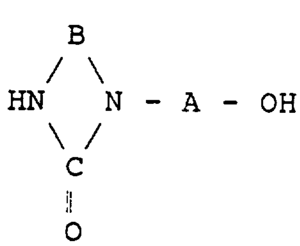

(III)

dans laquelle A et B ont les significations précitées, en présence d'un catalyseur constitué par un mélange formé (a) d'au moins un alcoolate de magnésium et (b) d'un composant choisi parmi les chélates de calcium avec les

composés 1,3-dicarbonylés, les dialkyloxydes d'étain, les dialkylalcoxydes d'étain et les dialkyldiesters d'étain.

2. Procédé selon la revendication 1, caractérisé par le fait qu'on utilise, comme constituant (a) du catalyseur, un alcoolate de magnésium $Mg(OR)_2$, R représentant un reste alkyle en $C_1$-$C_4$.

3. Procédé selon la revendication 2, caractérisé par le fait que R représente éthyle ou n-propyle.

4. Procédé selon l'une des revendications 1 à 3, caractérisé par le fait qu'on choisit, comme constituant (b) du catalyseur, un chélate de calcium avec un ester d'acide β-cétonique, comme l'ester acétylacétique, ou avec une 1,3-dicétone, comme l'acétylacétone, la 3-méthylacétylacétone, la benzoylacétone, le dibenzoylméthane, la 2,4-hexanedione, la 3,5-heptanedione, la 3-phénylacétylacétone, la 4,4,4-trifluoro-1-phényl-1,3-butanedione, la 2,2,6,6-tétraméthyl-3,5-heptanedione, la 1,1,1-trifluoro 5,5-diméthyl-2,4-hexanedione et la 1,1,1-trifluoro-2,4-pentanedione.

5. Procédé selon la revendication 4, caractérisé par le fait qu'on utilise, comme chélate de calcium, l'acétylacétonate de calcium.

6. Procédé selon l'une des revendications 1 à 5, caractérisé par le fait qu'on choisit le di-n-butyloxyde d'étain comme constituant (b) du catalyseur.

7. Procédé selon l'une des revendications 1 à 6, caractérisé par le fait que l'on utilise le constituant (a) du catalyseur dans une quantité de 0,5 à 4% en moles, le constituant (b), dans une quantité de 0,01 à 2% en moles, ces quantités étant données par rapport à l'alcool hétérocyclique de formule (III).

8. Procédé selon l'une des revendications 1 à 7, caractérisé par le fait que l'on conduit la réaction à une température comprise entre 75 et 105°C.

9. Procédé selon l'une des revendications 1 à 8, caractérisé par le fait que l'on utilise un rapport molaire du (méth) acrylate de formule (II) à l'alcool hétérocyclique de formule (III) qui est compris entre 1,1 et 7,0.

10. Procédé selon l'une des revendications 1 à 9, caractérisé par le fait que l'on conduit la réaction pendant une durée comprise entre 3 et 15 heures, sous une pression ne dépassant pas la pression atmosphérique.

11. Procédé selon l'une des revendications 1 à 10, caractérisé par le fait que l'on conduit la réaction en présence d'au moins un inhibiteur de polymérisation choisi parmi la phénothiazine, l'éther méthylique de l'hydroquinone, le ditertiobutylcatéchol, l'hydroquinone, le p-anilinophénol, la paraphénylène diamine et leurs mélanges en toutes proportions.

**Patentansprüche**

1. Verfahren zur Herstellung einer Verbindung der Formel

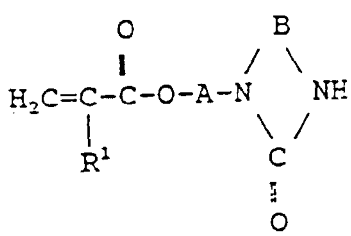

(I),

in der bedeuten:

- $R^1$ Wasserstoff oder Methyl,
- A und B unabhängig eine geradkettige oder verzweigte Kohlenwasserstoffkette mit 2 bis 5 Kohlenstoffatomen,

durch Umsetzung eines (Meth)acrylats der Formel

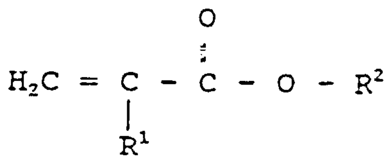 (II),

in der bedeuten:

- $R^1$ dasselbe wie weiter oben angegeben und
- $R^2$ eine $C_1$-$C_4$-Alkylgruppe

mit einem heterocyclischen Alkohol der Formel

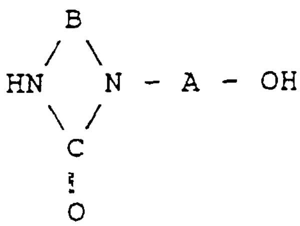 (III),

in der A und B dasselbe wie weiter oben angegeben bedeuten,
in Gegenwart eines Katalysators, der aus einem Gemisch besteht, das aus (a) mindestens einem Magnesiumalkoholat und (b) einem Bestandteil hergestellt wird, der unter den Chelaten von Calcium mit 1,3-Dicarbonyl-Verbindungen, den Dialkylzinnoxiden, Dialkylzinnalkoxiden und Dialkylzinndiestern ausgewählt ist.

**2.** Verfahren nach Anspruch 1, dadurch gekennzeichnet, daß als Bestandteil (a) des Katalysators ein Magnesiumalkoholat $Mg(OR)_2$ verwendet wird, worin A einen $C_1$-$C_4$-Alkylrest darstellt.

**3.** Verfahren nach Anspruch 2, dadurch gekennzeichnet, daß R Ethyl oder n-Propyl ist.

**4.** Verfahren nach einem der Ansprüche 1 bis 3, dadurch gekennzeichnet, daß als Bestandteil (b) des Katalysators ein Chelat von Calcium mit einem β-Ketocarbonsäureester, wie dem Acetylessigsäureester, oder mit einem 1,3-Diketon, wie Acetylaceton, 3-Methylacetylaceton, Benzoylaceton, Dibenzoylmethan, 2,4-Hexandion, 3,5-Heptandion, 3-Phenylacetylaceton, 4,4,4-Trifluor-1-phenyl-1,3-butandion, 2,2,6,6-Tetramethyl-3,5-heptandion, 1,1,1-Trifluor-5,5-dimethyl-2,4-hexandion und 1,1,1-Trifluor-2,4-pentandion, ausgewählt wird.

**5.** Verfahren nach Anspruch 4, dadurch gekennzeichnet, daß als Calciumchelat Calciumacetylacetonat verwendet wird.

**6.** Verfahren nach einem der Ansprüche 1 bis 5, dadurch gekennzeichnet, daß Di-n-butylzinnoxid als Bestandteil (b) des Katalysators verwendet wird.

**7.** Verfahren nach einem der Ansprüche 1 bis 6, dadurch gekennzeichnet, daß der Bestandteil (a) des Katalysators in einem Mengenanteil von 0,5 bis 4 Mol-% und der Bestandteil (b) in einem Mengenanteil von 0,01 bis 2 Mol-% verwendet wird, wobei diese Mengenanteile auf den heterocyclischen Alkohol der Formel (III) bezogen sind.

**8.** Verfahren nach einem der Ansprüche 1 bis 7, dadurch gekennzeichnet, daß die Umsetzung bei einer Temperatur von 75 bis 105 °C durchgeführt wird.

**9.** Verfahren nach einem der Ansprüche 1 bis 8, dadurch gekennzeichnet, daß ein Molverhältnis des (Meth)acrylats der Formel (II) zum heterocyclischen Alkohol der Formel (III) verwendet wird, das im Bereich von 1,1 bis 7,0 liegt.

**10.** Verfahren nach einem der Ansprüche 1 bis 9, dadurch gekennzeichnet, daß die Umsetzung während einer Dauer von 3 bis 15 h bei einem Druck, der nicht über Atmosphärendruck steigt, durchgeführt wird.

**11.** Verfahren nach einem der Ansprüche 1 bis 10, dadurch gekennzeichnet, daß die Umsetzung in Gegenwart mindestens eines Polymerisationsinhibitors durchgeführt wird, der unter Phenothiazin, Hydrochinonmethylether, Di-tert.-butylbrenzcatechin, Hydrochinon, p-Anilinophenol, p-Phenylendiamin und Gemischen dieser Inhibitoren in allen Mengenverhältnissen ausgewählt wird.

**Claims**

**1.** Process for the manufacture of a compound of formula:

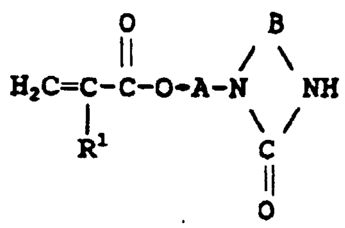

(I)

in which:

- $R^1$ represents hydrogen or methyl; and
- A and B each independently represent a straight or branched hydrocarbon chain having from 2 to 5 carbon atoms,

by reaction of one (meth)acrylate of formula:

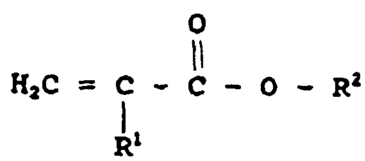

(II)

in which:

- $R^1$ has the abovementioned meaning; and
- $R^2$ represents a $C_1$-$C_4$ alkyl group,

with a heterocyclic alcohol of formula:

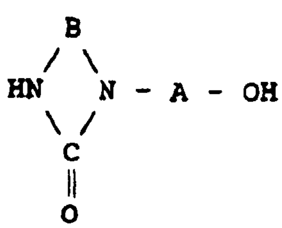

(III)

in which A and B have the abovementioned meanings,
in the presence of a catalyst consisting of a mixture formed (a) of at least one magnesium alkoxide and (b) of a component chosen from the chelates of calcium with 1,3-dicarbonyl compounds, dialkyltin oxides, dialkyltin alkoxides and dialkyltin diesters.

**2.** Process according to Claim 1, characterized in that a magnesium alkoxide $Mg(OR)_2$, R representing a $C_1$-$C_4$ alkyl

residue, is used as constituent (a) of the catalyst.

3. Process according to Claim 2, characterized in that R represents ethyl or n-propyl.

4. Process according to one of Claims 1 to 3, characterized in that a chelate of calcium with a β-keto acid ester, such as the acetylacetic ester, or with a 1,3-diketone, such as acetylacetone, 3-methylacetylacetone, benzoylacetone, dibenzoylmethane, 2,4-hexanedione, 3, 5-heptanedione, 3-phenylacetylacetone, 4,4,4-trifluoro-1-phenyl-1,3-butanedione, 2,2,6,6-tetramethyl-3,5-heptanedione, 1,1,1-trifluoro-5,5-dimethyl-2,4-hexanedione and 1,1,1-trifluoro-2,4-pentanedione is chosen as constituent (b) of the catalyst.

5. Process according to Claim 4, characterized in that calcium acetylacetonate is used as calcium chelate.

6. Process according to one of Claims 1 to 5, characterized in that di-n-butyltin oxide is chosen as constituent (b) of the catalyst.

7. Process according to one of Claims 1 to 6, characterized in that the constituent (a) of the catalyst is used in an amount of from 0.5 to 4 mol% and the constituent (b) is used in an amount of from 0.01 to 2 mol%, these amounts being given relative to the heterocyclic alcohol of formula (III).

8. Process according to one of Claims 1 to 7, characterized in that the reaction is performed at a temperature of between 75 and 105°C.

9. Process according to one of Claims 1 to 8, characterized in that a molar ratio of the (meth)acrylate of formula (II) to the heterocyclic alcohol of formula (III) which is between 1.1 and 7.0 is used.

10. Process according to one of Claims 1 to 9, characterized in that the reaction is performed for a period of between 3 and 15 hours, at a pressure which does not exceed atmospheric pressure.

11. Process according to one of Claims 1 to 10, characterized in that the reaction is performed in the presence of at least one polymerization inhibitor chosen from phenothiazine, hydroquinone methyl ether, di-tert-butylcatechol, hydroquinone, p-anilinophenol, paraphenylenediamine, and mixtures thereof in all proportions.